# EUROPÄISCHE PATENTSCHRIFT

(11) **EP 0 632 022 B1**
(45) Veröffentlichungstag und Bekanntmachung des Hinweises auf die Patenterteilung: **27.12.1996**
(21) Anmeldenummer: 94109543.2
(22) Anmeldetag: 21.06.1994
(51) Int. Cl.: C07C 319/14, C07C 323/24, C07C 323/29

(54) **Verfahren zur Herstellung von aminosubstituierten Thioethern**
Process for the preparation of amino-substituted thioethers
Procédé de préparation de thioéthers substitués par des groupes amine

(30) Priorität: 02.07.1993 DE 4322053; 28.09.1993 DE 4332978
(43) Veröffentlichungstag der Anmeldung: 04.01.1995
(73) Patentinhaber: BAYER AG, 51368 Leverkusen (DE)
(72) Erfinder: Kunde, Klaus, Dr., D-53819 Neunkirchen-Seelscheid (DE); Herd, Karl-Josef, Dr., D-51519 Odenthal (DE)

(56) Entgegenhaltungen:
- US-A- 3 950 542
- JOURNAL OF MEDICINAL CHEMISTRY Bd. 20, Nr. 11 , November 1977 Seiten 1357 - 1362 SOLOWAY, A.H. ET AL. 'Chemoimmunotherapy of Cancer. 2.'
- INORGANIC CHEMISTRY Bd. 25 , 1986 Seiten 4175 - 4180 VEIT, R. ET AL. 'Copper(II) and Nickel(II) Trinuclear Species with Dithiooxamide Derivative Ligands :: Structural, Magnetic, Spectroscopic and Electrochemical Properties'
- MONATSHEFTE FüR CHEMIE Bd. 110 , 1979 Seiten 767 - 789 ASINGER, F. ET AL. 'Zur Kenntnis der Reaktionsfähigkeit des unsubstituierten Thiomorpholins und alkylsubstituierter Thiomorpholine. 3. Mitteilung'

## Beschreibung

Die Erfindung betrifft ein neues Verfahren zur Herstellung von aminosubstituierten Thioethern. Derartige Verbindungen und ihre Herstellung sind grundsätzlich bekannt aus Journal of Medicinal and Chemistry, 1977, Vol. 20, Soloway, Chemo-Imuno-Therapie of Cancer, S. 1357 f.. Aus J.Org. Chem. 1992, 57, Poindexter, S. 6257-6265 ist weiterhin die Verwendung von 2-Oxazolidinonen zur Aminoethylierung aromatischer Verbindungen bekannt. Aus Journal of Medicinal and Chemistry, 1977, Vol. 20, Soloway, Chemo-Imuno-Therapie of Cancer, S. 1357 ist bekannt, Amino-substituierte Thioether durch Umsetzung von 2-Mercaptoethanol und 3-Bromethylaminohydrobromid herzustellen. Im Monatsheft für Chemie, Band 110 (1979), S. 767 bis 789, Asinger et al. wird β-Hydroxy-β'-aminodiethylsulfid aus 2-Mercaptoethanol und Ethylenimin erhalten. Das entsprechende β-Hydroxyethyl-γ-aminopropylsulfid wird in Organic Chemistry, Band 25, 1986, Seite 4175, Veit et al. durch Umsetzung von 3-Chlorpropylamin mit 2-Mercaptoethanol in Ethanol hergestellt. In US-A 3 950 542 werden derartige Verbindungen durch Umsetzung von Mercaptoalkylaminen mit Alkylenoxiden erhalten.

Die vorliegende Erfindung betrifft ein Verfahren zur Herstellung von Verbindungen der allgemeinen Formel (I) 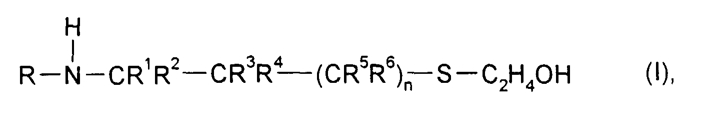 worin
- R, R¹-R⁶: unabhängig voneinander Wasserstoff, C₁-C₄-Alkyl, mit OH, C₁-C₄-Alkoxy oder SO₃H-substituiertes C₁-C₄-Alkyl, Phenyl, Benzyl, mit Halogen, C₁-C₄-Alkoxy oder SO₃H-substituiertes Phenyl oder Benzyl bedeuten und
- n: für 0 oder 1 steht,
dadurch gekennzeichnet, daß man 2-Mercaptoethanol mit Verbindungen der allgemeinen Formel (II) 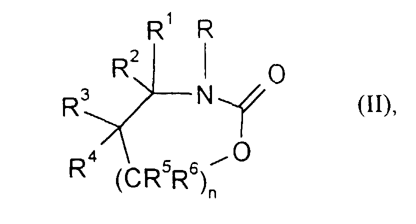 worin
- R, R¹-R⁶ und n: die oben angegebene Bedeutung besitzen,
umsetzt.

Die Umsetzung erfolgt vorzugsweise bei einer Temperatur von 50°C bis 250°C, vorzugsweise von 60°C bis 200°C.

Die Umsetzung kann gegebenenfalls in einen, Lösungsmittel erfolgen.

Als Lösemittel können alle die verwendet werden, deren pK_{S}-Wert deutlich geringer ist als der des 2-Mercaptoethanols, und die nicht selbst mit 2-Mercaptoethanol reagieren, z.B. Kohlenwasserstoffe, Ether, Alkohole, Carbonsäureamide, Sulfoxide, Sulfone; ausgenommen sind Amine.

Die Umsetzung von 2-Oxazolidinonen mit Thiophenolen ist zwar bekannt, mit Alkylmercaptanen ist dagegen keine Umsetzung zu den 2-(Alkylmercapto)-ethylaminen gelungen (G.S. Poindexter et al., J. Org. Chem. 1992, 57, 6257-6265).

Die Verbindungen der Formel (II) sind z.T. bekannt; ihre Herstellung erfolgt nach Verfahren, wie sie beschrieben sind in Houben-Weyl, Bd. E4 (1983) S. 192-211.

Geeignete Verbindungen der Formel II sind beispielsweise für n = 0
1-Oxo-1,3-oxazolidin (2-Oxazolidinon),
5-Methyl-2-oxo-1,3-oxazolidin,
3-Methyl-2-oxo-1,3-oxazolidin,
3-Phenyl-2-oxo-1,3-oxazolidin,
3-Benzyl-2-oxo-1,3-oxazolidin,
4-Phenyl-2-oxo-1,3-oxazolidin,
5-Phenyl-2-oxo-1,3-oxazolidin,
3,4-Diphenyl-2-oxo-1,3-oxazolidin,
3,5-Diphenyl-2-oxo-1,3-oxazolidin,
3,4-Dimethyl-5-phenyl-2-oxo-1,3-oxazolidin,
3-Phenyl-4-hydroxymethyl-2-oxo-1,3-oxazolidin,
3,5-Dimethyl-2-oxo-1,3-oxazolidin
3-Ethyl-2-oxo-1,3-oxazolidin,
4-Ethyl-2-oxo-1,3-oxazolidin,
und für n = 1
Tetrahydro[1,3]oxazin-2-on,
3-Methyl-tetrahydro[1,3]oxazin-2-on,
3-Phenyl-tetrahydro[1,3]oxazin-2-on,
3-Benzyl-tetrahydro[1,3]oxazin-2-on,
4-Methyl-tetrahydro[1,3]oxazin-2-on,
6-Methyl-tetrahydro[1,3]oxazin-2-on.

Bevorzugt wird das Verfahren zur Herstellung von Verbindungen der Formel (I), worin
- R: für Wasserstoff, Methyl, Ethyl, Phenyl oder Benzyl steht und
- R¹-R⁶: unabhängig voneinander Wasserstoff, Methyl und Phenyl bedeuten, insbesondere für Wasserstoff stehen.

Eine weitere bevorzugte Ausführungsform des Verfahrens ist dadurch gekennzeichnet, daß die Umsetzung von Verbindungen der Formel (II) mit 2-Mercaptoethanol und einer Base, vorzugsweise mit einer äquimolaren Menge Base, bezogen auf die Verbindung der Formel (II), erfolgt, worin R, R¹-R⁶ und n die obigen Bedeutungen haben. Als Base können beispielsweise Alkali, Alkalicarbonate, Alkalialkoholate oder tertiäre Amine, bevorzugt aber Natrium- bzw. Kaliumalkoholate, verwendet werden.

Die bevorzugte Temperatur für diese baseninduzierte Verfahrensvariante ist vorzugsweise 50 bis 150°C, insbesondere 60 bis 110°C, und als Lösungsmittel werden beispielsweise N-Methylpyrrolidon, Caprolactam, M-Methylcyprolactam, Cyclohexanol, Dimethylsulfoxid, Dimethylsulfon, Sulfolan, Methanol, Ethanol, n-Propanol, Isopropanol, n-Butanol, sec.-Butanol, Isobutanol, tert.-Butanol, Wasser oder Gemische davon eingesetzt, insbesondere Methanol, Ethanol, Isopropanol und/oder Wasser.

Diese baseninduzierte Verfahrensvariante ist ganz besonders bevorzugt für die Herstellung von Verbindungen der Formel (I), worin n = 0 bedeutet.

Desweiteren wird eine Ausführungsform zur Herstellung von Verbindungen der Formel (I), worin n = 0 und R und R¹-R⁴ die obengenannte Bedeutung haben, bevorzugt, die dadurch gekennzeichnet ist, daß man 2-Mercaptoethanol mit Verbindungen der Formel (II), worin n = 0 und R und R¹-R⁴ die obige Bedeutung haben, umsetzt.

Diese Verfahrensvariante erfolgt vorzugsweise bei einer Temperatur von 100 bis 250°C, insbesondere bei 150 bis 200°C, und kann in einem Lösungsmittel, vorzugsweise aber lösungsmittelfrei, durchgeführt werden. Als geeignete Lösungsmittel für das letztgenannte Verfahren können beispielsweise verwendet werden: Mesitylen, Naphthalin, 1-Methylnaphthalin, 2-Methylnaphthalin, Decalin, Tetralin, o-Dichlorbenzol, Nitrobenzol, Diphenyl, Diphenylether, 1-Hexanol, 1-Heptanol, 1-Octanol, 2-Octanol, Ethylenglykol, Diethylenglykol, Diethylenglykolmonomethylether, Diethylenglykolmonoethylether, Glycerin, N-Methylpyrrolidon, Caprolactam, N-Methylcaprolactam, Cyclohexanol, Dimethylsulfoxid, Dimethylsulfon, Sulfolan, insbesondere N-Methylpyrrolidon, Caprolactam und Sulfolan.

Die erfindungsgemäß hergestellten 2-(2'-Aminoalkylmercapto)-ethanole können verwendet werden als Zwischenprodukte für Pharmazeutika, Pestizide, insbesondere aber für Farbstoffe.

Besonders bevorzugt werden in dem erfindungsgemäßen Verfahren folgende Verbindungen der Formel (II) eingesetzt:
3-Phenyl-2-oxazolidinon, 3-Methyl-2-oxazolidinon, 2-Oxazolidinon, 3-Methyl-tetrahydro[1,3]-oxazin-2-on oder 3-Phenyl-tetrahydro[1,3]oxazin-2-on.

### Beispiel 1

78,1 g 2-Mercaptoethanol und 163 g 3-Phenyl-2-oxazolidinon werden unter N₂ 5 Stunden auf 160°C erhitzt. Man erhält 2-(2'-Phenylaminoethylmercapto)ethanol in fast quantitativer Ausbeute als schwach gelbliches Öl, dessen H-NMR-Spektrum folgende Signale aufweisen: (Lösemittel D₆-DMSO)
δ 2,58-2,75 ppm, 6H, m; δ 3,22 ppm, 2H, t; δ 4,80 ppm, 1H, s; δ 6,50-6,61 ppm, 3H, m; δ 7,05 ppm, 2H, d.

### Beispiel 2

156,2 g 2-Mercaptoethanol und 202 g 3-Methyl-2-oxazolidinon werden unter N₂ 5 Stunden auf 160°C erhitzt. Das 2-(2'-Methylaminoethylmercapto)ethanol wird unter vermindertem Druck destilliert. Man erhält ein farbloses Öl; Kp 5-6,5 mm = 124°C bis 130°C.

### Beispiel 3

78,1 g 2-Mercaptoethanol und 87 g 2-Oxazolidinon werden in 100 ml Diethylenglykolmonometyhlether 5 Stunden zum Sieden erhitzt. Man destilliert zunächst das Lösemittel und dann das 2-(2'-Aminoethylmercapto)ethanol unter vermindertem Druck; Kp 3 mm = 117°C bis 119°C.

### Beispiel 4

177 g 3-Phenyl-tetrahydro[1,3]oxazin-2-on werden in 200 ml Ethanol verrührt, mit 70 g Natriumethanolat und 80 g Mercaptoethanol versetzt und 7 Stunden unter Rückflußbedingungen erwärmt. Nach Reaktionsende wird das Ethanol abdestilliert, und der Rückstand mit 200 ml Wasser verrührt. Das ausgeschiedene schwach gelbliche Öl wird abgetrennt und als 2-(3'-Phenylamino-n-propylmercapto)ethanol charakterisiert.
¹H-NMR (D₆-DMSO): δ 1,8 ppm, 2H, m; δ 2,6 ppm, 4H, m; δ 3,09 ppm, 2H, q, δ 3,55 ppm, 2H, q; δ 4,8 ppm, 1H, t, δ 5,56 ppm, 1H, t, δ 6,54 ppm, 3H, m; δ 7,09 ppm, 2H, m.

### Beispiel 5

74 g N-Methyl-1,2-ethanolamin, 130 g Diethylcarbonat und 15 g Natriumethanolat werden in 50 ml Ethanol langsam auf 90°C erhitzt. Anschließend wird solange Ethanol abdestilliert, bis eine Sumpftemperatur von 110°C erreicht ist. Die warme Schmelze wird mit 72 g Mercaptoethanol versetzt und innerhalb von vier Stunden auf 160°C erwärmt. Das resultierende 2-(2'-(Methylamino)ethylmercapto)ethanol wird durch Destillation bei 140 bis 145°C/20 mm gereinigt.
¹H-NMR (D₆-DMSO/D₂O): δ 2,26 ppm, 3H, s; δ 2,52-2,65, 6H, m; δ 3,50, 2H, t.

## Patentansprüche

1. Verfahren zur Herstellung von Verbindungen der allgemeinen Formel (I) 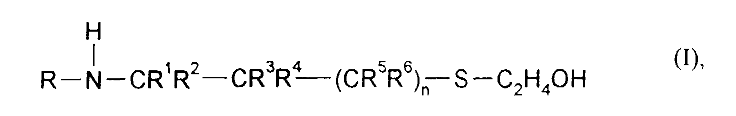 worin
R, R¹-R⁶ unabhängig voneinander Wasserstoff, C₁-C₄-Alkyl, mit OH, C₁-C₄-Alkoxy oder SO₃H-substituiertes C₁-C₄-Alkyl, Phenyl, Benzyl, mit Halogen, C₁-C₄-Alkoxy oder SO₃H-substituiertes Phenyl oder Benzyl bedeuten und
n für 0 oder 1 steht,
dadurch gekennzeichnet, daß man 2-Mercaptoethanol mit Verbindungen der allgemeinen Formel (II) 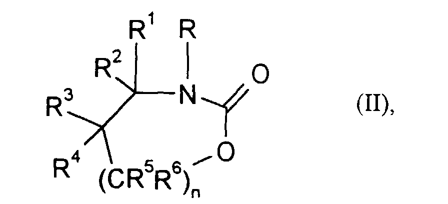 worin
R und R¹-R⁶ die oben angegebene Bedeutung besitzen,
umsetzt.

2. Verfahren nach Anspruch 1, dadurch gekennzeichnet, daß
R für Wasserstoff, Methyl, Ethyl, Phenyl oder Benzyl steht und
R¹-R⁶ unabhängig voneinander Wasserstoff, Methyl und Phenyl bedeuten.

3. Verfahren nach Anspruch 1, dadurch gekennzeichnet, daß die Substituenten R¹-R⁶ Wasserstoff bedeuten.

4. Verfahren nach Anspruch 1, dadurch gekennzeichnet, daß die Umsetzung mit einer Base, vorzugsweise äquimolare Mengen Base, bezogen auf die Verbindung der Formel (II), erfolgt.

5. Verfahren nach Anspruch 4, dadurch gekennzeichnet, daß die Umsetzung bei einer Temperatur von 50 bis 150°C, vorzugsweise von 60 bis 100°C erfolgt.

6. Verfahren gemäß Anspruch 1, dadurch gekennzeichnet, daß die Umsetzung mit Verbindungen der Formel (II) mit n = 0 erfolgt.

7. Verfahren gemäß Anspruch 6, dadurch gekennzeichnet, daß die Umsetzung bei einer Temperatur von 100 bis 250°C, vorzugsweise von 150 bis 200°C erfolgt.

8. Verfahren nach wenigstens einem der vorhergehenden Ansprüche, dadurch gekennzeichnet, daß als Verbindung der Formel (II) 3-Phenyl-2-oxazolidinon, 3-Methyl-2-oxazolidinon, 2-Oxazolidinon, 3-Methyl-tetrahydro[1,3]oxazin-2-on oder 3-Phenyl-tetrahydro[1,3]oxazin-2-on eingesetzt wird.

## Claims

1. Process for the preparation of compounds of the general formula (I) 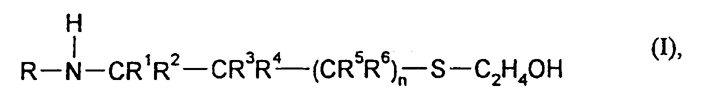 in which
R, R¹-R⁶ independently of one another denote hydrogen, C₁-C₄-alkyl, OH-, C₁-C₄-alkoxy-or SO₃H-substituted C₁-C₄-alkyl, phenyl, benzyl, or halogen-, C₁-C₄-alkoxy- or SO₃H-substituted phenyl or benzyl, and
n represents 0 or 1,
characterized in that 2-mercaptoethanol is reacted with compounds of the general formula (II) 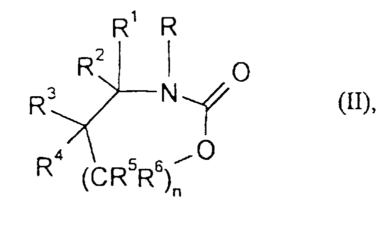 in which
R, R¹-R⁶ and n have the meaning given above.

2. Process according to Claim 1, characterized in that
R represents hydrogen, methyl, ethyl, phenyl or benzyl and
R¹-R⁶ independently of one another denote hydrogen, methyl and phenyl.

3. Process according to Claim 1, characterized in that the substituents R¹-R⁶ denote hydrogen.

4. Process according to Claim 1, characterized in that the reaction is carried out with a base, preferably equimolar quantities of base in relation to the compound of the formula (II).

5. Process according to Claim 4, characterized in that the reaction is carried out at a temperature from 50 to 150°C, preferably from 60 to 100°C.

6. Process according to Claim 1, characterized in that the reaction is carried out with compounds of the formula (II) where n = 0.

7. Process according to Claim 6, characterized in that the reaction is carried out at a temperature of from 100 to 250°C, preferably from 150 to 200°C.

8. Process according to at least one of the preceding claims, characterized in that the compound of the formula (II) which is employed is 3-phenyl-2-oxazolidinone, 3-methyl-2-oxazolidinone, 2-oxazolidinone, 3-methyl-tetrahydro[1,3]oxazin-2-one or 3-phenyltetrahydro[1,3]oxazin-2-one.

## Revendications

1. Procédé pour la préparation des composés de formule générale I 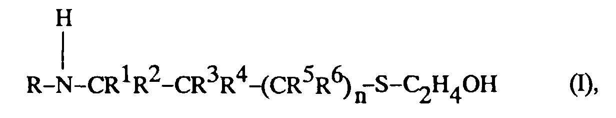 dans laquelle
R, R¹ à R⁶ représentent chacun, indépendamment les uns des autres, l'hydrogène, un groupe alkyle en C₁-C₄, un groupe alkyle en C₁-C₄ substitué par des groupes OH, alcoxy en C₁-C₄ ou SO₃H, un groupe phényle, benzyle, un groupe phényle ou benzyle substitué par des halogènes, des groupes alcoxy en C₁-C₄ ou SO₃H, et n est égal à 0 ou 1,
caractérisé en ce que l'on fait réagir le 2-mercaptoéthanol avec des composés de formule générale II 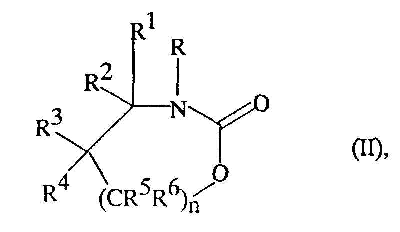 dans laquelle
R et R¹ à R⁶ ont les significations indiquées ci-dessus.

2. Procédé selon la revendication 1, caractérisé en ce que R représente l'hydrogène, un groupe méthyle, éthyle, phényle ou benzyle et R¹ à R⁶ représentent chacun, indépendamment les uns des autres, l'hydrogène, un groupe méthyle ou phényle.

3. Procédé selon la revendication 1, caractérisé en ce que les symboles R¹ à R⁶ représentent l'hydrogène.

4. Procédé selon la revendication 1, caractérisé en ce que la réaction est effectuée en présence d'une base, de préférence en quantité équimoléculaire par rapport au composé de formule II.

5. Procédé selon la revendication 4, caractérisé en ce que la réaction est réalisée à une température de 50 à 150°C, de préférence de 60 à 100°C.

6. Procédé selon la revendication 1, caractérisé en ce que la réaction est réalisée avec des composés de formule II dans laquelle n = 0.

7. Procédé selon la revendication 6, caractérisé en ce que la réaction est effectuée à une température de 100 à 250°C, de préférence de 150 à 200°C.

8. Procédé selon au moins une des revendications qui précèdent, caractérisé en ce que l'on met en oeuvre en tant que composé de formule II la 3-phényl-2-oxazolidinone, la 3-méthyl-2-oxazolidinone, la 2-oxazolidinone, la 3-méthyl-tétrahydro[1,3]oxazine-2-one ou la 3-phényl-tétrahydro[1,3]oxazine-2-one.
